# EUROPÄISCHE PATENTSCHRIFT

(11) **EP 0 626 821 B1**
(45) Veröffentlichungstag und Bekanntmachung des Hinweises auf die Patenterteilung: **25.09.1996**
(21) Anmeldenummer: 93902058.2
(22) Anmeldetag: 28.01.1993
(51) Int. Cl.: A61B 5/042, A61B 17/36

(54) **HERZKATHETER**
CARDIAC CATHETER
CATHETER CARDIAQUE

(30) Priorität: 21.02.1992 DE 4205336
(43) Veröffentlichungstag der Anmeldung: 07.12.1994
(73) Patentinhaber: GSF-Forschungszentrum für Umwelt und Gesundheit, GmbH, D-85764 Oberschleissheim (DE)
(72) Erfinder: WEBER, Helmut, D-8027 Neuried (DE)
(86) Internationale Anmeldenummer: DE9300069
(87) Internationale Veröffentlichungsnummer: WO9316634

(56) Entgegenhaltungen:
- EP-A- 0 144 764
- DE-C- 3 718 139
- FR-A- 2 639 237
- The American Journal of CARDIOLOGY 54:186-192; 1984
- Circulation 71:579-586; 1985

## Beschreibung

Die Erfindung betrifft einen Herzkatheter nach dem Oberbegriff des Patentanspruches 1.

Es ist bekannt, einen Herzkatheter mit einer Sondenspitze derart auszubilden, daß mit ihm über Elektroden subendokardiale Areale geortet werden können (Am J. Cardiol 54: 186-192; 1984). Die Position der Sondenspitze bleibt jedoch aufgrund von Herzbewegungen nicht über längere Zeiten definiert, so daß eine Zuordnung von Diagnose zu einer zeitlich nachgeordneten Therapie nicht gewährleistet ist.

Andererseits gibt es Herzkatheter, mit denen über eine innerhalb des Katheterschlauches verlegte Laserfaser Photoablationen von Herzgewebe durchgeführt werden (Circulation 71: 579 bis 586; 1985). Bei diesen Herzkathetern ist nicht auszuschließen, daß die Laserfaser mit dem Endokard (der Herzinnenhaut) direkt in Berührung kommt und eine Photoissektion des Subendokards und Myokards bzw. eine Herzwandperforation verursachen.

Desweiteren ist aus der DE 37 18 139 C1 ein Katheter der gattungsgemäßen Art bekannt, bei welchem die Spülung in arteriellen Bereichen des Herzens wegen der dort herrschenden Turbulenzen nicht ausreicht.

Die Erfindung hat die Aufgabe einen Herzkatheter der e. g. Art derart auszubilden, daß in allen Bereichen des Herzens eine ausreichende Spülung mit einer physiologischen Lösung ermöglicht wird, wobei der übrige Aufbau vereinfacht werden soll.

Gelöst wird diese Aufgabe durch das kennzeichnende Merkmal des Patentanspruches 1.

Die übrigen Ansprüche geben vorteilhafte Weiterbildungen und Ausführungsformen der Erfindung an.

Der erfindungsgemäße Herzkatheter ermöglicht die Bestrahlung ausgewählter subendokardialer Zonen, ohne daß die Querzfaserspitze das Endokard berührt und ohne daß sich die Katheterspitze verlagern kann; die Bildung eines Blutgerinnsels während der Laserbestrahlung wird ebenfalls vermieden. Dies wird durch das erfindungsgemäß gestaltete distale Schlauchende erzielt, in das Fühler eingebaut sind und aus dem die Querzfaser definiert in Bezug auf die Herzinnenhaut ausgefahren werden kann. Dadurch wird die Katheterspitze an das Endokard fixiert und gleichzeitig die Spitze der Glasfaser vom Endokard auf den erforderlichen Abstand gehalten. Das Blut wird durch kontinuierliche Einspritzung von physiologischer Kochsalzlösung durch den Katheter entlang der Querzfaser weggespült, so daß während der Bestrahlung zwischen der Faserspitze und der Herzinnenhaut ein nicht koagulierbares Medium vorliegt.

Durch die trichterförmig ausgebildete Folie 8 wird der Blutstrom weitgehend vom Bestrahlungsraum ferngehalten, so daß ein Spülmittelstrom von etwa 1 ml/sec. für eine störungsfreie Bestrahlung ausreicht.

Mit Hilfe des Katheters können z. B. Herzrhythmusstörungen geheilt werden, ohne Operation am offenen Herzen, ohne die Gefahr des intrakardialen Elektroschocks, ohne Perforationsgefahr des Herzens, ohne Thrombose- und Emboliegefahr und ohne Vollnarkose. Auch eine evtl. lebenslange Medikamenteneinnahme mit den bekannten Nebenwirkungen und Risiken, oder der Einbau kostspieliger Elektrostimulatoren wie z. B. Herzschrittmacher, Kardiovertor/Defibrillator, können vermieden und folglich eine deutliche Verbesserung der Lebenqualität der Patienten und eine erhebliche Kosteneinsparung erzielt werden. Der Katheter kann auch in anderen Anwendungsbereichen eingesetzt werden, wie z. B. als Thermo- oder als Mikrowellensonde.

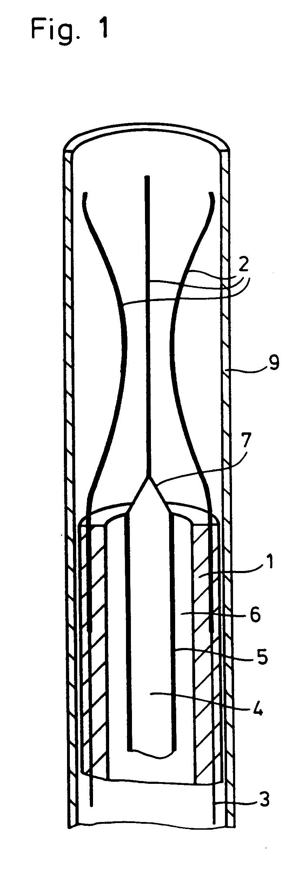

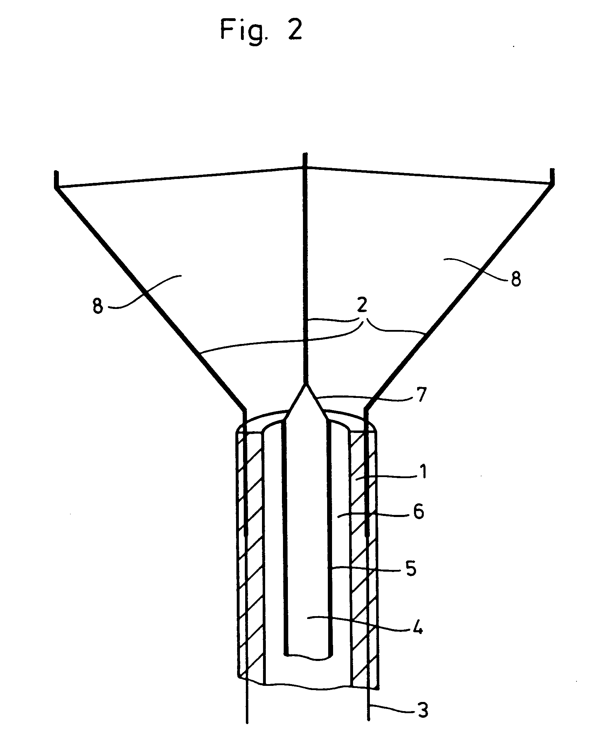

Die Erfindung wird im folgenden anhand eines Ausführungsbeispiels mittels der Fig. 1 und 2 näher beschrieben.

Dabei zeigt die Figur 1 den Katheter in einem Führungskatheter 9 (Schleuse) und die Fig. 2 die distale Katheterspitze mit gespreizten Elektroden.

In Fig. 1 ist das (in eine Schleuse/Führungskatheter 9 eingeführte) distale Ende eines Herzkatheters im Schnitt dargestellt. Es handelt sich um das Ende eines Schlauches 1 aus Kunststoff dessen Spitze, von einer Elektrodenanordnung 2 gebildet wird. Die Elektroden sind in das distale Schlauchende eingelassen und fest verankert gegeneinander elektrisch getrennt und geben ihre elektrischen Potentiale über Kabel 3 an eine nicht dargestellte Auswerteeinheit ab. Innerhalb des Schlauches 1 ist koaxial die Faser 4 gelegt, deren Oberfläche mit einer Ummantelung 5 versehen ist. Die Faser kann im Innern 6 des Schlauches 1 axial geführt werden. Führungselement sind hier nicht dargestellt. Am distalen Ende des Schlauches 1 sind vorzugsweise drei der als Fühler 2 ausgebildete Elektroden befestigt. Sie bestehen aus Metalldrähten, die durch Formung eine bestimmte Vorspannung erhalten haben. Die Vorspannung und die Länge der Fühler 2 sind derart gewählt, daß sie einerseits die Spitze 7 der Faser 4 zentrieren, andererseits sich nach außen hin ausspreizen können, wenn das distale Ende des Katheters aus der Schleuse (Führungskatheter) 9 zum Herzen vorgeschoben wird. Durch das Aufspreizen der Fühler, wird eine zwischen den Fühlern angebrachte Folie 8 (Membran) ausgespannt (diese Folie ist in Fig. 2 dargestellt und der Übersichtlichkeithalber hier weggelassen), die den Abstand zwischen den gespreizten Fühlern stabilisiert und durch die Ummantelung des Raumes zwischen der Faserspitze 7 und dem zu bestrahlenden Endokardareal, diesen von starker Blutdurchströmung schützt. Die Folie ist auch am distalen Schlauchende befestigt, so daß sie im gespreizten Zustand der Fühler einen zum Endokard hin offenen Trichter bildet.

Die Fig. 2 zeigt die distale Katheterspitze ohne den Führungskatheter 9.

Die Fühlerenden stehen dann über der Spitze 7 der Faser 4, so daß diese nicht mit dem Endokard in Berührung kommt. Die Fühlerenden selbst verankern sich beim Anlegen an das Endokard (Vorschieben des Herzkatheters) und sorgen somit für eine Fixierung/Stabilisierung des distalen intrakardialen Endes des Schlauches 1 bzw. der Faser 4.

Zwischen der Faser 4 bzw. Isolation 5 und der Schlauchinnenwand im Hohlraum 6 fließt eine physiologische Lösung (z. B. Kochsalzlösung), die zum Schlauchende hinausströmt. Hierbei kühlt sie die Faserspitze und hält den Raum am Schlauchende und den Raum um die Faser 4 bis hin zu dem zu bestrahlenden Gewebe blutfrei. Gleichzeitig verhindert die Lösung das Eindringen von Blut in das Kathetersystem und die Entstehung von Blutgerinnseln im Strahlungsbereich.

Bei einem Außendurchmesser der Ummantelung 5 von 0,7 mm sollte der Innendurchmesser des Schlauches etwa 1 mm betragen.

## Patentansprüche

1. Herzkatheter (9) in Form eines Schlauches (1) mit als Fühler ausgebildeten Elektroden (2), wobei die Fühler unter einer nach außen gerichteten Vorspannung stehen und mit einer axial im Schlauch (1) liegenden Faser (4), wobei zwischen Schlauch (1) und Faser (4) Raum (6) für die Zufuhr von Spülflüssigkeit bleibt, gekennzeichnet durch eine Folie (8), welche an dem Schlauch (1) und an den Fühlern (2) derart befestigt ist, daß sie im gespreizten Zustand der Fühler (2) einen nach oben offenen Trichter bildet.

2. Herzkatheter nach Anspruch 1, dadurch gekennzeichnet, daß die Fühler (2) im distalen Ende des Schlauches (1) gehaltert sind.

3. Herzkatheter nach Anspruch 1 oder 2, dadurch gekennzeichnet, daß elektrische Zuleitungen (3) zu den Fühlern (2) im Schlauch (1) verlaufen.

4. Herzkatheter nach einem der Ansprüche 1 bis 3, dadurch gekennzeichnet, daß zwischen der Innenwand des Schlauches (1) und der Faser (4) ein freier schlauchförmiger Zwischenraum bleibt.

5. Herzkatheter nach einem der Ansprüche 1 bis 4, dadurch gekennzeichnet, daß die Faser eine Lichtleitfaser ist.

## Claims

1. Intercardiac catheter (9) having the form of a hose (1) with electrodes (2) made as sensors, the sensors prestressed towards the outside and a fiber (4) running axially in the hose (1), a space (6) left between the hose (1) and the fiber (4) for supply of rinsing liquid, characterized by a foil (8) fixed to the hose (1) and the sensors (2) such that in the expanded state of the sensors (2) they make up a funnel open on top.

2. Intercardiac catheter as claimed in 1, characterized by the sensors (2) fixed at the distal end of the hose (1).

3. Intercardiac catheter as claimed in 1 or 2, characterized by electric leads (3) to the sensors (2) routed in the hose (1).

4. Intercardiac catheter as claimed in one of claims 1 to 3, characterized by a hose-shaped gap remaining between the inner wall of the hose (1) and the fiber (4).

5. Intercardiac catheter as claimed in one of claims 1 to 4, characterized by the fiber being an optical fiber.

## Revendications

1. Cathéter cardiaque (9) sous la forme d'un tuyau (1) avec des électrodes (2) réalisées en capteurs, dans lequel les capteurs se trouvent sous une prétension dirigée vers l'extérieur et avec une fibre (4) située axialement dans le tuyau (1), tandis qu'entre le tuyau (1) et la fibre (4), il reste un espace (6) pour l'amenée de liquide de nettoyage,
caractérisé par
une feuille (8), qui est fixée sur le tuyau (1) et sur les capteurs (2), de manière qu'elle forme à l'état écarté des capteurs (2) un entonnoir ouvert vers le haut.

2. Cathéter cardiaque selon la revendication 1,
caractérisé en ce que
les capteurs (2) sont maintenus à l'extrémité distale du tuyau (1).

3. Cathéter cardiaque selon les revendications 1 ou 2,
caractérisé en ce que
des conduites électriques (3) se développent dans le tuyau vers les capteurs (2).

4. Cathéter cardiaque selon une des revendications 1 à 3,
caractérisé en ce que
entre la paroi interne du tuyau (1) et la fibre (4) il reste un espace intermédiaire libre en forme de tuyau.

5. Cathéter cardiaque selon une des revendications 1 à 4,
caractérisé en ce que
la fibre est une fibre optique.
